# EUROPEAN PATENT APPLICATION

(11) **EP 1 221 313 A2**
(43) Date of publication of application: **10.07.2002**
(21) Application number: 01830787.6
(22) Date of filing: 20.12.2001
(51) Int. Cl.: A61K 7/06, A61K 7/32, C11D 7/32, C11D 7/44

(54) **Salts of undecylenoyl glutamate or of undecylenoyl hydrolyzate of wheat or rice proteins in detergent or cosmetic compositions**

(30) Priority: 09.01.2001 IT TO010006; 07.11.2001 IT TO011054
(71) Applicant: Zschimmer & Schwarz Italiana S.p.A., 13038 Tricerro (Vercelli) (IT)
(72) Inventor: Ariotto, Angelo, 15030 Terruggia (Alessandria) (IT); Guala, Fabrizio, 13039 Trino (Vercelli) (IT); Merlo, Elisabetta, 13039 Trino (Vercelli) (IT); Villa, Giovanni, 20030 Paderno Dugnano (Milano) (IT)
(74) Representative: Rambelli, Paolo

(57) **Abstract**

The invention describes detergent or cosmetic compositions having hydrating and preservative properties and simultaneously anti-dandruff and/or anti-odour properties. These compositions comprise a salt of undecylenoil glutamate and/or undecylenoil hydrolyzate of wheat and/or rice proteins. Since such salts are able to perform also a preservative and hydrating effect the compositions of the invention do no need the addition of further preservative or hydrating agents or, at most, contain concentrations thereof which are not efficacious per se.

## Description

The present invention relates to a detergent or cosmetic composition having anti-dandruff and/or anti-odour properties, simultaneously provided with hydrating and preservative properties.

In the formulation of cosmetic or detergent compositions with anti-dandruff and/or anti-odour properties it is known to use derivatives of undecylenic acid. However, often the problem of commonly utilised derivatives is that of excessive delipidation of the corneal layer of the skin acting as a solvent on the functional lipids contained in it and simultaneously to subtract the water soluble substances present on it. All this causes excessive drying of the skin and fragility of the hair, which are problems commonly resolved by adding to the formulations suitable hydrating agents.

In the formulation of detergent and cosmetic compositions in general it is moreover necessary to add preservative agents which have the function of preserving the composition from pollution by bacteria and other micro-organisms, such as, for example fungi and yeasts, which can be the cause of infections in man. However, the disadvantage associated with the use of preservative agents is that these to be efficacious must often be utilised in concentrations which are irritating or sensitising for the tissue with which they come into contact.

The present invention therefore has the purpose of providing a detergent or cosmetic composition provided with the functional properties indicated above (that is to say, anti-dandruff and/or anti-odour), which does not have the above mentioned disadvantages.

This object has been achieved by the present inventors, who have identified derivatives of undecylenic acid which, when formulated in a detergent or cosmetic composition, are not only able to perform an anti-dandruff and/or anti-odour activity, but are simultaneously also able to confer on the said composition hydrating and preservative properties.

These derivatives are salts of undecylenoil glutamate and/or undecylenoil hydrolyzate of wheat and/or rice proteins.

The salts of undecylenoil glutamate can be obtained by the Schotten-Bauman reaction by causing the amino group of the glutamic acid to react with chloride of undecylenoil in a basic environment, according to the following reaction diagram:

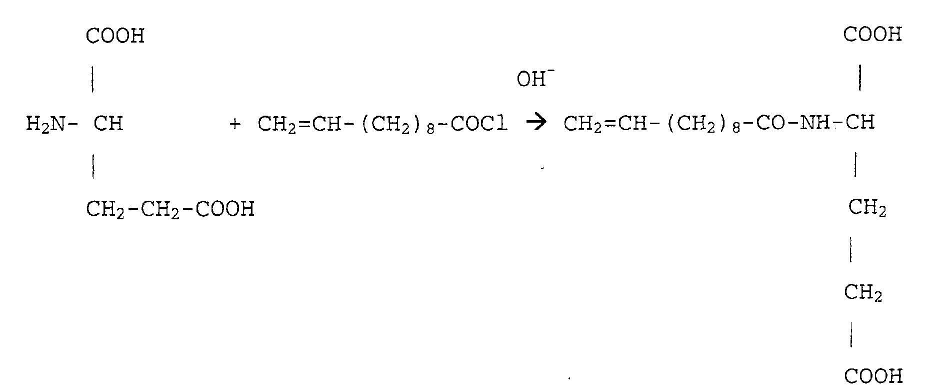

In the same way, the salts of undecylenoil hydrolyzate can be obtained by the Schotten-Bauman reaction by making the amino groups present in the hydrolyzate react with undecylenoil chloride in a basic environment, according to the following reaction diagram:

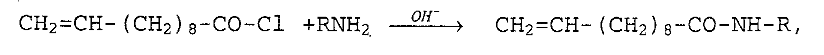

Where R is the peptidic residue which can be obtained by hydrolysis of rice and/or wheat proteins.

The peptidic residue (or hydrolyzate) can be obtained by acid hydrolysis (in the presence of HCl) of gluten (wheat) and/or husks (rice) and subsequent filtration to separate the product from the dregs.

As an alternative to chemical hydrolysis an hydrolysis of enzymatic type can also be utilised, exploiting the amylase and protease enzymes.

The product obtained is a mixture of peptides having a molecular weight between 1000 and 4000 Dalton. Within the ambit of the present description this product will sometimes be indicated as "peptidic residue" and at other times as "proteic hydrolyzate" or "hydrolyzate of wheat and/or rice proteins".

During the Schotten-Bauman reaction the dissociated carboxylic groups present are neutralised by salification with a base chosen in dependence on the desired salt, for example NaOH or KOH. Alternatively, to obtain salts of a weak base such as, for example, TEA, the sodium or potassium salt is brought to a pH of 2.0-3.0 with phosphoric acid to obtain the acid form and subsequently dissolved in water and neutralised with the desired base.

In the above-described salts the dissociated carboxylic groups are preferably neutralised with cations selected from the group which consists of: cations belonging to the group of alkaline metals or other monovalent cations such as Cu⁺; cations belonging to the group of alkaline earth metals or other bivalent cations such as Pb²⁺; trivalent cations such as Al³⁺; polyvalent cations such as Sn⁴⁺; NH₄⁺ or aminic bases selected from triethanol amine, monoethanol amine, diethanol amine, monoisopropanol amine, triisopropanol amine, 2-aminobutanol, aminoethyl propandiol, arginine, lisine, ornithine, aminomethyl propanol, aminomethyl propandiol and 2-amino-2-hydroxymethyl-1,3-propandiol. Such neutralising cations can be utilised also in combinations with one another.

The present invention is therefore directed to the use of a compound which is a salt of undecylenoil glutamate and/or undecylenoil hydrolyzate of wheat and/or rice proteins as a preserving and hydrating agent and simultaneously as an anti-dandruff and/or anti-odour agent, in the formulation of a detergent or cosmetic composition free from other preservative or hydrating agents, or including other preservative and/or hydrating agents at a non-efficacious concentration and/or one insufficient to confer the desired characteristics of preservability and/or hydration in a similar or corresponding composition free from the said compound.

The present invention is also directed to a detergent or cosmetic composition provided with hydrating and preservative properties and simultaneously anti-dandruff and/or anti-odour properties, comprising, as anti-dandruff and/or anti-odour agent a compound which is a salt of undecylenoil glutamate and/or undecylenoil hydrolyzate of wheat and/or rice proteins, the said compound further having preservative and hydrating effect, the said composition being thus free from further preservative and/or further hydrating agents, or including other preservative and/or hydrating agents at non-effective concentrations and/or concentrations insufficient to confer the desired characteristics of preservability and/or hydration in a corresponding or similar composition free from the said compound.

The expression "corresponding or similar composition" is intended to indicate a composition constituted by the same components in the same quantities as the composition of the invention, but which is differentiated from this by the fact that the salts of undecylenoil glutamate and/or undecylenoil hydrolyzate of wheat and/or rice proteins is substituted with an equal quantity of another anionic surfactant agent.

In general this can mean that a detergent or cosmetic composition has the desired characteristics of preservability when it responds positively to the test for the evaluation of the efficacy of the preserving power. Such tests are conducted according to the indication provided by the US pharmacopaeia (USP) and or by the Cosmetic, Toiletry and Fragrance Association (CTFA). These methods, and other similar methods, involve the inoculation of the samples with micro-organisms of different types and the aerobic microbic count of these at different times to evaluate the level of survival.

The preserving efficacy of the salts of undecylenoil glutamate and proteic undecylenoil hydrolyzate indicated above has been confirmed by means of the challenge tests described in examples 1 and 2.

The tests, as well as confirming the preserving effacacy of the salts, have further shown that they increase with a reduction in pH.

Consequently, it is preferred that the cosmetic or detergent composition according to the invention has a pH less than or equal to 6, still more preferably a pH in the range of 4 to 6.

The use of the above-indicated salts in the formulation of detergent or cosmetic compositions thus allows the elimination or in any event the substantial reduction of the concentration of the usual preservative agents, to a concentration which, if present, will nevertheless be less than the irritant and/or sensitising activity threshold.

Among the preservative agents commonly utilised in detergent or cosmetic compositions of the prior art, but absent in the composition according to the invention, or at most present in non-irritant and non preserving concentrations, there are for example formaldehyde, chlorine, hypochlorite, compounds releasing chlorine, chlorine dioxide, iodine and iodophor, phenol, cresol, chlorocresol, amphoteric compounds with preservative characteristics, chlorexidine, peracetic acid and dihydroacetic acid, compounds of mercury, alcohol, sorbic acid, benzoic acid, salicylic acid, boric acid, formic acid, propionic acid and salts of these derivatives, bronopol, 5-bromo-5-nitrodioxane heaxamethylenetetra amine, DMDM hydantoin, various hydantoins such as MDM hydantoin, chloracetamide, ureas such as urea imidazolidinyl urea diazolidinyl, inorganic sulphites, trichlosane, parabenes, isothiazolinones, usnic acid and its salts, chlorophene, hexachlorophene, dichlorophene, bromochlorophene, trichlorocarbon, chlorofluorocarbon, benzamidine classified as preservatives, amines classified as preservatives, dimethyl oxazolidine, ethylbicyclooxazolidine, dimethyl hydroxymethyl pirazol, polyamino propyl biguanide, hydroxy methyl glycinate of sodium, methyl dibromoglutaronitrile, glycaryl monolaurate and their mixtures.

The non-irritant and non-preservative concentration varies in dependence on the compound considered, and can be easily determined by the man skilled in the art since the above-listed compounds are all known and usually utilised in cosmetics as preservatives.

In general it can be seen that a detergent or cosmetic composition has the desired characteristics of hydration when it is capable of obviating the symptoms and manifestations of dry hair and skin (according to the definition of "hydrating compound" in A short textbook of cosmetology, K.F. De Polo, first edition 1998).

The salts of undecylenoil glutamate and proteic undecylenoil hydrolyzate have such hydrating properties. The molecules of undecylenoil glutamate and of proteic undecylenoil hydrolyzate once in contact with the skin in fact split up into their components of proteic hydrolyzate and glutamate on one side and fatty acid on the other.

Both the hydrolyzed proteins of wheat and rice and glutamic acid have the hydrating property.

The hydrolyzed proteins of wheat and rice in contact with the skin in fact form a continuous protective layer over the corneal layer thereof protecting it from external aggressive agents and increasing the hydration by reduction of the TEWL (Trans Epidermal Water Loss). Moreover such hydrolyzed proteins are a source of amino acids which constitute 40% of the physiological hydrating factor (NMF).

The glutamic acids on the other hand enters into a series of biological cycles contributing to the correct cutaneous hydration and to maintenace of the natural cutaneous acidity thanks to the two carboxylic groups. It in fact constitutes 15% of the keratin and goes as far as forming part of the E (γ-glutamyl) lisine bonds present in the protein casing and keratoline which constitutes the cellular membrane of the corneocity. It can be transformed into PCA, one of the components of NMF, that is to say a set of water soluble substances responsible for the correct hydration, as well as into proline and hydroxyproline (these two amino acids are fundamental for the synthesis of collagen and elastine). The acylglutamates moreover act as selective solvents for the main components of the sebum, or the squalene, therefore leaving the functional lipids of the corneal layer which control hydration unaltered.

The undecylenic acid component instead will perform its peculiar anti-dandruff and deodorant action.

The use of salts of undecylenoil glutamate and/or undecylenoil hydrolyzate of proteins of wheat and/or rice in the formulation of the detergent or cosmetic compositions according to the invention thus make it possible to eliminate or at any rate substantially to reduce the addition of hydrating factors, emollients or palliatives for the skin, the concentration of which, if present, will however be less than the efficacy threshold for hydrating activity.

Among hydrating agents commonly utilised in detergent or cosmetic compositions of the prior art but absent in the composition according to the invention, or at most present in non-efficacious concentrations, there are for example glycerine, sorbitol, glycol propylene, polyethylene glycol with the molecular weight from 200 to 600, Sorbeth-30, urea, lactic acid and its salts, mucopolysaccharids such as ialuronic acid and condroit in sulphate, orothic acid, lanolin, petrolatum, mineral oils, occlusive substances which hydrate the skin by preventing the evaporation of water, and their mixtures.

The concentrations which are not efficacious for hydration vary in dependance on the compound considered, and can be easily determined by the man skilled in the art since the compounds listed above are all known and usually utilised in cosmetics as hydrating agents.

Detergent or cosmetic compositions according to the invention preferably include an aqueous medium, even if the salts used can be equally used in emulsions as primary hydrophilic emulsifiers.

The total concentration of the salts of undecylenoil glutamate and/or undecylenoil hydrolyzate of wheat and/or rice proteins in the compositions according to the invention is preferably a concentration which, itself, is sufficient to confer the characteristics of preservability, activity and desired hydration, or else such as to confer the said characteristics in combination with other preserving agents and/or hydrating agents present in concentrations themselves not sufficient to confer such characteristics.

This concentration is preferably between 2% and 25% by weight of active substance with respect to the total composition, more preferably between 5% and 15% by weight of active substance. In this range of concentrations, in fact, the salts present are able correctly to perform all their functions.

Detergent or cosmetic compositions according to the invention can moreover include at least one further and separate surfactant agent chosen from ionic surfactants such as, for example, alkyl sulphate salts and alkyl ether sulphate salts (for example salts of sodium, magnesium, TEA, MEA and iammonium), salts of starch/starch ether sulphates, salts of alkyl semisulphosuccinates and of alkyl sulphosuccinates, salts of alkyl ether semisulphosuccinates and of alkyl ether sulphosuccinates, salts of acyl amido semisulphosuccinates and of acyl amido sulphosuccinates, salts of dodecyl benzene sulphonic acid, salts of alkyl/alkyl ether sulpho acetates, salts of organic sulphonate molecules and/or sulphamate (for example, α-olefin sulphonate), salts of alkyl/alkyl ether carboxylate, alkyl phosphonate, esters of phosphoric acid, salts of acyl septionates; amphoteric surfactants such as, for example, alkyl betaine, alkyl amedopropylbetaine, oxides of amine and of starch, amphocarboxy acetates and amphocarboxy diacetates, amphocarboxy pionates and amphocarboxy dipropionates; non-ionic surfactants such as, for example, various amides, ethoxylated and non-ethoxylated fatty amines, ethoxylated non-iphenyl, APG (alkyl polyglucosides), AEG (alkyl ethoxyglucosides), esters/ethers of fatty acids with glycerol and/or ethoxylated and non-ethoxylated sugars, various ethoxylated/propoxylated esters and non ethoxylated/propoxylated esters, ethoxylated/propoxylated and non-ethoxylated/propoxylated fatty acids, silicone molecules; cationic surfactants containing for example at least one atom of quaternary N.

Some products in which the compositions of the invention can be used are: shampoo, foam baths, foam showers, washing up liquid, liquid soaps, solid soaps, lotions, emulsions of various nature, balsams, products having simultaneously detergent and conditioning effect on the skin and/or the hair, oils, emollient and detergent milks, face cream and/or cream for the body and/or hair, detergents for intimate hygiene, bryliantine, solutions for permanent waves, hair colouring, dentifrice, medicated cosmetics, pharmaceutical products.

These examples are provided exclusively for the purpose of illustration and are not intended to limit the scope of the invention in any way.

### EXAMPLES

### Example 1a: Test on the preservative efficacy of wheat proteic undecylenoil hydrolyzate.

To evaluate the preservative efficacy of wheat protein undecylenoil hydrolyzate there was conducted a challenge test on an aqueous solution of undecylenoil hydrolyzate of wheat proteins in the form of a sodium salt at 10 % of active material; the pH of the solution was 5.

They were prepared for the four mixed inoculations illustrated in Table 1 (bacteria gram plus; bacteria gram minus; yeast; filamentary fungi) and each of these was inoculated with a sample of the above indicated solution.

**Table 1**

| | | |
|---|---|---|
| BACTERIA GRAM+ | 1x10⁷ | Staphylococcus aureus ATCC6538 Staphylococcus epidermidis ATCC 12228 |
| BACTERIA GRAM- | 1x10⁷ | Escherichia coli ATCC 8739 Pseudomonas aeruginosa ATCC 9027 Enterobacter cloacae ATCC 13047 Pseudomonas putida (from cosmetics) |
| YEASTS | 1x10⁷ | Candida albicans ATCC 10231 Saccharomyces cerevisiae ATCC 9763 |
| FILAMENTARY FUNGI | 1x10⁵ | Aspergillus niger ATCC 16404 Penicillium funiculosum ATCC 9644 |

The challenge test consists in monitoring the survival of the inoculations over time; such survival is indicative of the capacity for preservation of the cosmetic product during the period of use by the consumer.

In particular, the survival of the inoculations was tested after 24 hours, 7 days and 28 days from inoculations. The test at 24 hours represents a datum for the purpose of evaluating the speed of the preservative system, that at 7 days indicates however the degree of risk of pollution of the product whilst the test at 28 days, as well as representing a confirmation of the previously obtained data, makes it possible to put in evidence the possible formation of resistance strains.

The results obtained are illustrated in the following Table 2.

**Table 2**

| Values obtained in the survival tests of the inoculations expressed in units forming colonies per gram of product (u.f.c./g). | | | | |
|---|---|---|---|---|
| Survival | Gram + (u.f.c./g) | Gram - (u.f.c./g) | Fil. fungi (u.f.c./g) | Yeasts (u.f.c./g) |
| 24 hours | < 10 | 10² | < 10 | < 10 |
| 7 days | < 10 | < 10 | < 10 | < 10 |
| 28 days | < 10 | < 10 | < 10 | < 10 |

By means of successive dilutions there was therefore calculated the minimum inhibiting concentration (MIC) of an aqueous solution at pH5 of sodium salt wheat protein undecylenoil hydrolyzate against some micro-organisms involved in the aetiology of dandruff and some skin diseases and the like. The results obtained are illustrated in the following Table 3.

**Table 3**

| MICROORGANISM | MIC (%) |
|---|---|
| Pytyrosporum Ovale* | 2,5% |
| Malassezia Furfur* | 3,5% |
| Ephidermophyton^{o} | 3,5% |
| Trichophyton Mentagrophite^{o} | 3,5% |

| | |
|---|---|
| *Agent involved in the aetiology of dandruff and skin disorders. | |
| ^{o} Agent involved in the aetiology of many diseases of the skin and the like. | |

### Example 1b: Test on the preserving efficacy of undecylenoil glutamate

The preserving efficacy of the molecule of undecylenoil glutamate was tested according to the procedure described in example 1a. The challenge test was conducted on an aqueous solution of undecylenoil glutamate in the form of sodium salt at 1.5% of active material; the pH of the solution was 5. The results obtained are substantially equivalent to those illustrated in example 1a in relation to sodium salt undecylenoil hydrolyzate.

### Example 2: Hydrating face gel free from preservatives

Rice protein sodium undecylenoil hydrolyzate (30% active material): 9%.
Laurylethoxy sulphate of ethoxylated sodium 3.7 moles (25% active material): 30%
Lauramide propyl betaine (30% active material): 4%
Sodium cocoamphodiacetate (26% active material): 3%
PEG-7 glyceryl cocoate (100% active material): 0.5%
Citric acid: enough to bring pH to 5.0
NaCl, colour, perfume and water: to 100%

### Example 3: Shower gel with hydrating effect

Sodium undecylenoil hydrolyzate of wheat protein (30% active material): 8%
Sodium lauryl sulpho acetate (65% active material): 1.5%
Laurylethoxysulphate of ethoxylated sodium 3 moles (25% active material): 40%
Cocoamedopropyl betaine (30% active material): 10%
Disodiumlaurylethoxysemisulphosuccinate (30% active material): 6%
Citric acid: enough to bring pH to 4.5
NaCl, colour, perfume and water: to 100%

### Example 4: Preparation for foot hygiene

Potassium undecylenoil hydrolyzate of wheat protein (30% active material): 5%
Laurylethoxysulphate of ethoxylated 3 mole sodium (25% active material): 30%
Lauramidepropyl betaine (30% active material): 4%
Laurylsulphate of triethanol amine (39% active material):3%
Citric acid: enough to bring pH to 4.2
NaCl, colour, perfume and water: to 100%

### Example 5: Anti-dandruff shampoo

Potassium undecylenoil hydrolyzate of rice protein (30% active material): 4%
Lauramidepropyl betaine (30% active material): 20%
Laurylethoxysulphate of ethoxylated 2 mole sodium (25% active material): 20%
Cocoa starch (100% active material): 2%
Laurylsulphate of monoethanol amine (27% active material): 5%
Citric acid: enough to bring pH to 5.0
NaCl, colour, perfume and water: to 100%

### Example 6: Grease normalising face wash

Potassium undecylenoil hydrolyzate of wheat protein (30% active material): 10%
Sodium lauryl sulphoacetate (65% active material): 6%
Citric acid: enough to bring pH to 5.5
Colour, perfume, excipients and water: to 100%

### Example 7: Aftershave lotion in emulsion form

Lypo phase:
   Potassium undecylenoil hydrolyzate of wheat protein hydrolyzate (30% active material): 6%
   Rice oil: 10%
   Almond oil: 0.5%
   Glycerile monostearate: 4%
   Alcohol cetylstearyl 2.5%
Water phase:
   Citric acid: enough to bring pH to 5.0
   Colour, perfume, excipients and water: to 100%
   Xanthan gum: 0.2%

### Example 8: Sports shower gel with antimicotic effect

Potassium undecylenoil glutamate (30% active material): 12%
Laurylethoxysulphate of ethoxylated sodium 3 moles (25% active material): 30%
Sodium cocoamphodiacetate (26% active material): 15%
Laurylsulphate of triethanol amine (39% active material): 6%
Citric acid: enough to bring pH to 4.5
NaCl, colour, perfume and water: to 100%

### Example 9: Anti-dandruff shampoo

Sodium undecylenoil glutamate (30% active material): 16%
Laurylethoxysulphate of 3 mole ethoxylated sodium (27% active material): 40%
Sodium cocoamphodiacetate (26% active material): 10%
Laurylsulphate of triethanol amine (39% active material): 5%
Cocoa starch (100% active material): 1%
Citric acid: as required
NaCl, colour, perfume and water: to 100%

### Example 10: Preparation for intimate hygiene

Sodium undecylenoil glutamate (30% active material): 5%
Laurylethoxysulphate of 2 mole ethoxylated magnesium (25% active material): 25%
Oxide of cocoaamidepropyl betaine (30% active material): 4%
Cocoa starch: 1%
Citric acid: enough to bring pH to 4.0
NaCl, colour, perfume and water: to 100%

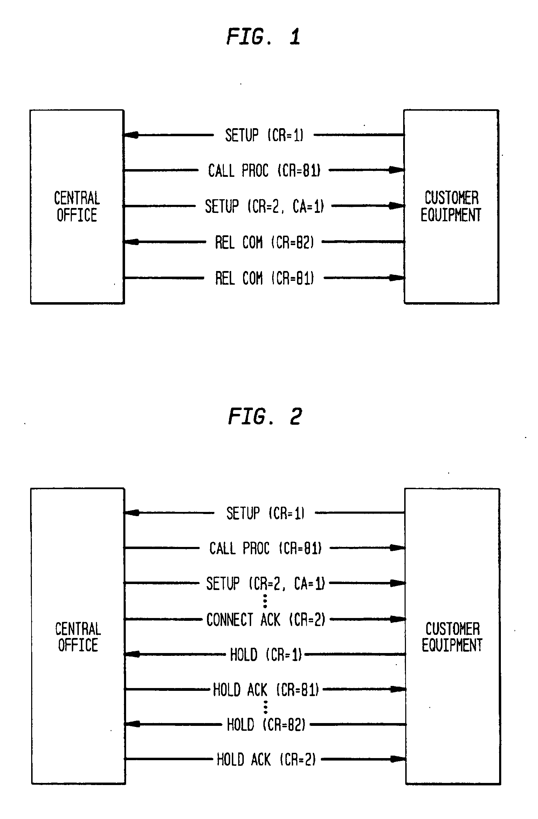

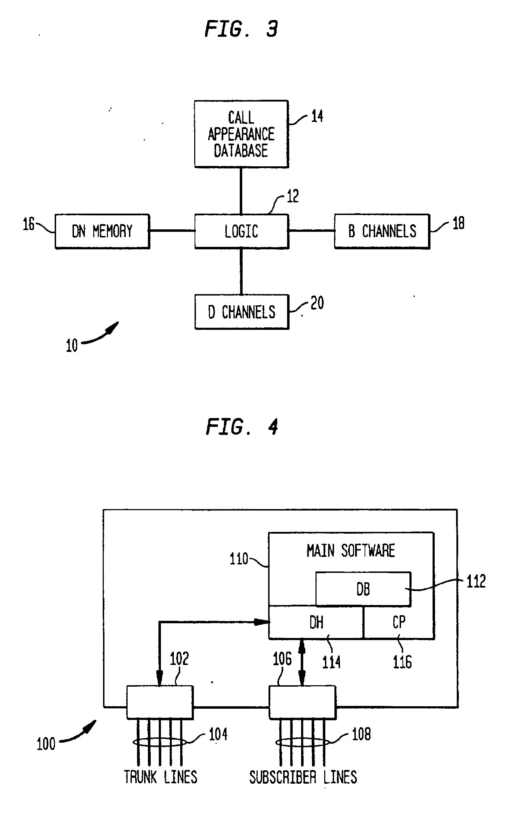

## Claims

1. A detergent or cosmetic composition having hydrating and preservative properties and simultaneously anti-dandruff and/or anti-odour properties, comprising as anti-dandruff and/or anti-odour agent a salt of undecylenoil glutamate and/or undecylenoil hydrolyzate of wheat and/or rice proteins, the said salts also having preservative and hydrating effect, the said composition thus being free from further preservatives and further hydrating agents, or comprising other preservative and/or other hydrating agents at non-effective concentrations and/or insufficient to confer the preservability characteristics and/or desired hydration in a similar or analagous composition free from the said salts.

2. A composition according to Claim 1, in which the said salt is chosen from the group consisting of: salts with cations of alkaline metals or other monovalent cations such as Cu⁺; salts with cations of alkaline earth metals or other bivalet cations such as Pb²⁺; salts with trivalent cations such as Al³⁺; salts with polyvalent cations such as Sn⁴⁺; salts with NH₄⁺ or with an amine base chosen from triethanol amine, monoethanol amine, diethanol amine, monoisopropanol amine, triisopropanol amine, 2-amino butanol, amino ethyl propandiol, arginine, lisine, olythine, amino methyl propanol, amino methyl propandiol, 2-amino-2-hydroxymethyl-1,3-propandiol; and their combinations.

3. A composition according to Claim 1 or Claim 2, further comprising an aqueous medium.

4. A composition according to any of Claims 1 to 3, having a pH less than or equal to 6.

5. A composition according to any of Claims 1 to 4, in which the said further hydrating agent is chosen from the group consisting of: glycerine, sorbitol, glycol, propylene, polyethylene glycol with molecular weight from 200 to 600, Sorbeth-30, urea, lactic acid and its salts, mucopolysaccarides such as ialuronic acid and condroytinsulphate, orothic acid, lanolin, petrolatum, mineral oils, occlusive substances which hydrate the skin by preventing the evaporation of water, and their mixtures.

6. A composition according to any of Claims from 1 to 5, in which the said further preservative agent is chosen from the group which consists of: formaldehyde, chlorine, hypochlorite, compounds releasing chlorine, chlorine dioxide, iodine and iodophors, phenol, cresol, chlorocreasol, amphoteric compounds with preservative characteristics, chlohexadine, paracetic and dihydroacetic acid, compounds of mercury, alcohols, sorbic acid, benzoic acid, salicylic acid, boric acid, formic acid, propionic acid and salts derived from these, bromopol, 5-bromo-5-nitrodioxane, hexamethyline tetramine, DMDM hydantoine, various hydantoines such as MDM hydantoine, chloacetamide, ureas such as imidazolidinyl urea and diazolidinyl urea, inorganic sulphites, triclosane, parabenes, isothyazolenones, uznic acid and its salts, chlorophene, hexachlorophene, dichlorophene, bromochlorophene, trichlorocarbon, chlorofluorocarbon, benzamydene classified as preservatives, amines classified as preservatives, dimethyloxyazolidine, ethylbicycloxyazolidine, dimethylhydroxymethylpirazol, polyaminopropylbiguanide, hydroxymethylglycilate of sodium, methyl dibromoglutaronitrile, glycerile monolaurate and their mixtures.

7. A composition according to any of Claims from 1 to 6, comprising further and separate surfactant agents chosen from an ionic surfactants, cationic surfactants, non-ionic surfactants, amphoteric surfactants and their mixtures.

8. A composition according to Claim 7, in which the said further and separate surfactant agent is chosen from sodium laurylsulphate, sodium laurylethoxysulphate and their mixtures.

9. A composition according to any of Claims from 1 to 8, in which the concentration of the said salt is in the range from 2 to 25% by weight of active material with respect to the total weight of the composition.

10. A composition according to Claim 9, in which the said concentration is in the range from 5 to 15% by weight of active material.

11. Use of a salt of undecylenoil glutamate and/or undecylenoil hydrolyzate of wheat and/or rice proteins as preservative agent and hydrating agent and simultaneously anti-dandruff and/or anti-odour agents in the formulation of a detergent or cosmetic composition as defined in any preceding claim.
